Europäisches Patentamt

European Patent Office

Office européen des brevets

(19)

(11) Veröffentlichungsnummer: **0 312 907 B1**

## (12) EUROPÄISCHE PATENTSCHRIFT

(45) Veröffentlichungstag der Patentschrift: **12.01.94**

(21) Anmeldenummer: **88116972.6**

(22) Anmeldetag: **13.10.88**

Die Akte enthält technische Angaben, die nach dem Eingang der Anmeldung eingereicht wurden und die nicht in dieser Patentschrift enthalten sind.

(51) Int. Cl.5: **G01N 33/544**, G01N 33/545, G01N 33/548, //G01N33/78

(54) **Immunreaktives Trägermaterial.**

(30) Priorität: **22.10.87 DE 3735684**

(43) Veröffentlichungstag der Anmeldung:
**26.04.89 Patentblatt 89/17**

(45) Bekanntmachung des Hinweises auf die Patenterteilung:
**12.01.94 Patentblatt 94/02**

(84) Benannte Vertragsstaaten:
**AT BE CH DE ES FR GB GR IT LI LU NL SE**

(56) Entgegenhaltungen:
EP-A- 0 063 810
EP-A- 0 185 372
EP-A- 0 197 784

ZELLSTOFF, PAPIER, 5. Auflage, VEB Fachbuchverlag, Leipzig (DE), 1979; S. 233-235&NUM;

(73) Patentinhaber: **BOEHRINGER MANNHEIM GMBH**

**D-68298 Mannheim(DE)**

(72) Erfinder: **Mangold, Dieter, Dr.**
**Hüttenmüllerstrasse 33**
**D-6701 Maxdorf(DE)**
Erfinder: **Nötzel, Siegfried, Dr.**
**Silbergasse 38**
**D-6901 Wilhelmsfeld(DE)**
Erfinder: **Lerch, Rolf**
**Lessingstrasse 4**
**D-6804 Ilvesheim(DE)**
Erfinder: **Jering, Helmut, Dr. rer. nat.**
**Anton-Bartl-Strasse 4**
**D-8132 Tutzing(DE)**

EP 0 312 907 B1

**Beschreibung**

Die Erfindung betrifft ein immunreaktives poröses Trägermaterial und Verfahren zu seiner Herstellung. Immunreaktive poröse Trägermaterialien spielen in verschiedenen Zweigen der Technik eine wichtige Rolle. Beispielsweise werden derartige Materialien für analytische und präparative Verfahren benötigt, bei welchen ein Partner einer Immunreaktion an einen unlöslichen Träger fixiert eingesetzt wird. Die Fixierung des Partners der Immunreaktion an den unlöslichen Träger kann durch chemische oder physikalische Kräfte erfolgen.

So sind seit langem Methoden zur Herstellung kovalenter Bindungen zwischen einem festen Trägermaterial und einer daran zu bindenden chemischen Substanz, wie insbesondere auch biologisch aktiven Proteinen, bekannt. Ein Nachteil dieser Methoden besteht generall darin, daß sie eine chemische Veränderung des biologisch aktiven Materials bewirken, die in vielen Fällen auch eine Veränderung der biologischen Aktivität zur Folge hat.

Eine weitere bekannte Methode ist die Einschlußpolymerisation derartiger biologisch aktiver Substanzen. Hier bleibt zwar das Molekül als solches in der Regel unverändert und behält daher auch seine biologische Wirksamkeit unverändert bei. Die Zugänglichkeit für den anderen, in einer flüssigen Phase vorliegenden Partner der Immunreaktion ist jedoch drastisch beschränkt.

Daher wurde vielfach auf das dritte bekannte Verfahren zurückgegriffen, welches die Nachteile der kovalenten Bindung und der Einschlußpolymerisation vermeidet, nämlich die absorptive Fixierung löslicher Substanzen an einem geeigneten Trägermaterial. Diese Methode wiederum hat jedoch den Nachteil, daß die Bindung an den festen Träger wesentlich schwächer ist als bei den beiden zuerst erwähnten Methoden.

Spezielle Probleme ergaben sich hinsichtlich der Haftfestigkeit bei einer adsorptiven Bindung, wenn poröse Trägermaterialien angewendet werden sollen. Aus der US-A 3,888,629 ist eine Methode bekannt, die dieses Problem verringert, indem die Fixierung in den Poren des Trägermaterials durch Ablaufenlassen einer Präzipitationsreaktion zwischen den beiden Partnern einer Immunreaktion, also zwischen Antikörper und Antigen oder Hapten, durchgeführt wird. Bei dieser Methode werden die beiden Lösungen der jeweiligen Partner der Immunreaktion rasch gemischt und dann sofort das poröse Trägermaterial damit imprägniert. In der WO 82/02601 wird eine Variante dieser Methode beschrieben. Man imprägniert hierzu das poröse Trägermaterial mit einer Lösung des ersten Partners der Immunreaktion und behandelt danach mit einer Lösung des zweiten Partners der Immunreaktion, so daß die Immunreaktion selbst im porösen Trägermaterial ablaufen kann unter permanenter Fixierung des gewünschten immunreaktiven Stoffes ohne chemische Veränderung oder Nachteilen hinsichtlich seiner Zugänglichkeit für andere Reaktionspartner.

Diese Methode der Bindung eines immunreaktiven Stoffes auf einem porösen Trägermaterial verringert zwar die oben beschriebenen Probleme, weist jedoch einen anderen Nachteil auf, nämlich eine ungleichmäßige Verteilung der fixierten immunreaktiven Substanz auf dem Trägermaterial. Dieser Nachteil ist besonders dann gravierend, wenn dieses immunreaktive poröse Trägermaterial z. B. für Zwecke der quantitativen Analyse niedrig konzentrierter Haptene oder Proteine eingesetzt werden soll. Die Forderung nach Stabilität des Antigen-Antikörper-Netzwerkes sowie nach niedrigen Gleichgewichts-Dissoziations-Konzentrationen der immunreaktiven Partner aus den Immunkomplexen relativ zur Konzentration der zu bestimmenden Haptene und Proteine läßt sich nur erreichen, wenn der verwendete Antikörper eine hohe Affinität zum jeweiligen Antigen besitzt. Erfahrungsgemäß setzt die Präzipitatbildung beim Mischen derartig immunreaktiver Partner spontan ein. Bei der Herstellung technischer Mengen eines immunreaktiven porösen Trägermaterials durch Imprägnieren eines porösen Trägermaterials mit kurz zuvor gemischten Lösungen der immunreaktiven Partner kommt es erwartungsgemäß zu zeitlich und räumlich unkontrollierbarer Präzipitatbildung auf dem Trägermaterial.

Die quantitative Dosierung des Immunosorbens erfolgt zweckmäßigerweise durch Ausschneiden einer bestimmten Papierfläche bei einem immunreaktiven Papier, durch Auswahl einer bestimmten Anzahl von Kügelchen bei einem kugelförmigen porösen Trägermaterial oder durch Ausweigen einer bestimmten Menge des immunreaktiven Trägermaterials. Bei ungleichmäßiger Verteilung des fixierten immunreaktiven Stoffes scheidet eine derartige einfache Dosierungsmethode jedoch aus.

Ein weiterer Nachteil der bekannten Immunpräzipitationstechnick besteht darin, daß sehr hochgereinigte Antigene benötigt, was eine Vorreinigung durch Immunosorption und damit eine aufwendige Herstellungsweise nötig macht.

Eine gleichmäßige Verteilung des Immunkomplex-Präzipitats im Trägermaterial wird erreicht durch das "homogene" Verfahren, das in der DE-A-34 46 636 beschrieben ist. Danach wird ein poröses Trägermaterial mit einer Lösung der beiden Partner der Immunreaktion getränkt, die zur Verhinderung der Präzipitation mit einem Hemmstoff versetzt ist. Durch Entfernen des Hemmstoffs, z. B. durch Trocknen des getränkten Vlieses, wird die Präzipitation eingeleitet. Falls die Immunpartner nicht im Verhältnis des Heidelberger-

2

Maximums, bei dem die entstandene Trübung maximal wird, zusammengegeben wurden, muß der Überschuß des einen Immunpartners durch Waschen des Trägermaterials abgespült werden. Dabei werden auch Immunkomplexe, die nicht fest adsorbiert sind sowie leicht dissoziierende Immunkomplexe vom Trägermaterial abgewaschen. Werden die Immunpartner im Heidelberger-Maximum zur Präzipitation so ist zwar ein zusätzlicher Waschschritt zur Entfernung eines Überschusses des einen Immunpartners nicht mehr nötig. Nicht fest gebundene sowie leicht dissoziierende Immunkomplexe verbleiben dabei allerdings auf dem porösen Trägermaterial und können zu Störungen führen. Verwendet man beispielsweise ein in vorstehender Weise hergestelltes immunreaktives Trägermaterial für die quantitative Bestimmung eines immunreaktiven Analyten, so wird der im Test für den Analyten gemessene Konzentrationswert verfälscht, da durch Auswaschen von für die Messung oder für eine Meßreaktion relevanten Bestandteilen in das Meßmedium ein Leervert für die Messung resultiert. Je nach Meßmethode führt dies dann zu einem zu hohen oder zu niedrigen Wert für die zu ermittelnde Analytkonzentration.

Aufgabe der vorliegenden Erfindung war es, ein immunreaktives poröses Trägermaterial bereitzustellen, bei dem diese Nachteile vermieden werden, insbesondere bei dem die Immunkomplexe fester an dem Trägermaterial haften.

Gelöst wird diese Aufgabe durch ein immunreaktives, poröses Trägermaterial, bestehend aus einem porösen Trägermaterial und darauf präzipitierten Immunkomplexen, dadurch gekennzeichnet, daß das poröse Trägermaterial aus Fasern besteht und mit einem oder mehreren Naßfestmitteln behandelt ist.

Ebenfalls Gegenstand der Erfindung ist ein Verfahren zur Herstellung eines immunreaktiven porösen Trägermaterials durch Präzipitieren von Immunkomplexen auf ein poröses Trägermaterial, dadurch gekennzeichnet, daß das poröse Trägermaterial, das aus Fasern besteht, mit einem oder mehreren Naßfestmitteln behandelt wird und darauf anschließend die Immunkomplexe präzipitiert werden.

Das oder die Naßfestmittel können - falls gewünscht - schon dem Faserbrei zugesetzt werden, das poröse Trägermaterial wird anschließend getrocknet und darauf werden die Immunkamplexe präzipitiert.

Wesentliches Merkmal der Erfindung ist die Verwendung eines im Endpunkt der Verarbeitung nicht oder nur in sehr geringem Maß in Wasser löslichen Stoffes, der die Fasern des ursprünglichen Trägermaterials umgibt. Solche Stoffe sind insbesondere die gelegentlich bei der Papierherstellung benutzten Naßfestmittel.

Sie sorgen für bessere mechanische Belastbarkeit von Papier. Die im allgemeinen gegenüber der Trockenfestigkeit erniedrigte Naßfestigkeit wird erhöht (Zellstoff, Papier, 5. Auflage, VEB Fachbuchverlag Leipzig 1979, Seite 233 - 235). Natürlich wird im allgemeinen auch die Trockenfestigkeit gegenüber unbehandelten Papieren erhöht.

Überraschend war nun, daß die Verwendung von Naßfestmitteln erhebliche Vorteile für die mit ihrer Hilfe hergestellten immunreaktiven porösen Trägermaterialien bewirken.

Zur Herstellung von Trägermaterialien, die mit Naßfestmitteln behandelt sind, finden die bei der Papierherstellung üblichen Methoden und Gerätschaften Verwendung, falls im folgenden nichts anderes angegeben wird.

Erfindungsgemäß sind als faserige Ausgangsstoffe für die Herstellung von mit Naßfestmitteln behandelten Trägermaterialien Gemische aus Zellulosefasern verschiedener Herkunft und Beschaffenheit, vorzugsweise Sulfitzellstoff, regenerierte Zellulose, Zellwolle oder Linters, mit Fasern künstlicher Polymere, vorzugsweise allgemein üblicher Polyester, Polyamide und Polyacrylnitrile, geeignet. Der Mengenanteil dieser Kunststoffasern an der Gesamtmasse wird durch ihre Verarbeitbarkeit begrenzt. Mit allgemein üblichen Papiermaschinen konnten beispielsweise Trägermaterialien mit bis zu 95, bevorzugt bis zu 60 Gewichts-% Kunststoffaseranteil hergestellt werden. Den restlichen Anteil stellen Zellulosefasern der oben angegebenen Art.

Die Länge der Fasern hat für die Erfindung keine wesentliche Bedeutung. Als vorteilhaft haben sich jedoch Fasern mit einer Länge von 1 - 6 mm erwiesen.

Auf in der Papierherstellung geläufige Art wird aus den Fasern ein Faserbrei angerichtet, der die Fasern gegebenenfalls nach Zerkleinern in der gewünschten Länge enthält.

Diesem Faserbrei werden nun ein oder mehrere Naßfestmittel zugesetzt. Gebräuchliche Naßfestmittel sind Epichlorhydrinharze, Melaminformaldehydharze, Harnstofformaldehydharze, Polyethylenimine und spezielle Carboxymethylcellulosen. Diese können in dem erfindungsgemäßen Verfahren eingesetzt werden. Zur Verwendung im Sinne der Erfindung sind auch Stoffe der oben angegebenen Substanzklassen geeignet, die zwar selbst keine überlegenen Naßfesteigenschaften aufweisen, jedoch durch ihre ähnliche strukturelle Beschaffenheit ähnliche Wirkung im Sinne der Erfindung haben.

Als besonders bevorzugt haben sich die Handelsprodukte Luresin[R],Tylose[R], Madurit[R], Etadurin[R] und Urecoll[R] erwiesen. Bei Luresin[R] handelt es sich um ein Polyamidoamin-Epichlorhydrinharz, bei Tylose[R] um eine Carboxymethylcellulose, bei Madurit[R] um ein Melaminformaldehydharz, Etadurin[R] ist ein Polyamidoa-

EP 0 312 907 B1

min-Epichlorhydrinharz und Urecoll^R (BASF AG) ist ein Harnstoff-Formaldehydharz. Erfindungsgemäß kann auch ein Gemisch von Naßfestmitteln der oben angegebenen Art oder äquivalenter Verbindung verwendet werden.

Der Anteil der Naßfestmittel im Faserbrei ist abhängig von dem jeweils verwendeten Naßfestmittel, beträgt aber vorteilhaft 0.05 - 6 Gew.-%, vorzugsweise 2 - 4 Gew.-%, bezogen auf die verwendete Menge an Zellulosefasermaterial. Natürlich kann auch mehr Naßfestmittel zugegeben werden; das führt jedoch nicht zu einer deutlichen Verbesserung im Sinn der Erfindung.

Gewünschtenfalls können auch noch andere Hilfsstoffe in den Faserbrei eingebracht werden, wie zum Beispiel Härter, Binder, Verfestiger, Pigmentstoffe oder Leim.

Auf übliche Weise wird aus dem Faserbrei in der Papiermaschine das poröse Trägermaterial hergestellt. Dabei beeinflußt die Ablaufgeschwindigkeit der Flüssigkeit die Dichte des entstehenden porösen Trägermaterials. Durch Nachpressen kann die Dichte des Trägermaterials noch erhöht werden.

Im darauf folgenden Trocknungsvorgang, der bei erhöhter Temperatur durchgeführt werden kann, werden die mit Naßfestmittel behandelten porösen Trägermaterialien gegebenenfalls auch gealtert. Dazu gehört beispielsweise im Fall der Melaminformaldehydharze, die als wasserlösliche Vorkondensate gehandelt werden, die Weiterführung bzw. Beendigung der Kondensation.

Die nach dem beschriebenen Verfahren erzeugten, mit Naßfestmittel behandelten, porösen Trägermaterialien sollen eine Dichte von 0.2 -0.5 $g/cm^3$, vorzugsweise von 0.3 - 0.4 $g/cm^3$, aufweisen. Andernfalls werden die getrockneten, mit Naßfestmittel behandelten, porösen Trägermaterialien kalandriert. Alle Trägermaterialien, die eine solche Dichte aufweisen, haben Hohlräume zwischen den sie aufbauenden Fasern und sind somit porös.

Die Herstellung eines mit Naßfestmittel behandelten Trägermaterials kann jedoch auch auf andere Art erfolgen. Eine weitere bevorzugte Ausführung besteht darin, daß das Naßfestmittel nicht schon dem Faserbrei zugegeben wird, sondern erst das ohne dessen Zugabe in der Papiermaschine fertiggestellte Trägermaterial beispielsweise in einer Imprägniermaschine mit einer Lösung des Naßfestmittels getränkt wird. Dabei beträgt die Konzentration des Naßfestmittels in der Lösung 0.01 - 0.6 Gew.-%, vorzugsweise 0.35 - 0.45 Gew.-%. Anschließend wird das mit Naßfestmittel behandelte poröse Trägermaterial bei Temperaturen von 50 -150°C, vorzugsweise 80 - 100 °C bis zu einer Restfeuchte von 2 bis 6 Gew. %, vorzugsweise 3 Gew. % getrocknet.

Der Fachmann kennt weitere geeignete Möglichkeiten, mit Naßfestmitteln behandelte Trägermaterialien herzustellen (z. B. trocken verlegte Vliese).

Die mit Naßfestmitteln behandelten Trägermaterialien weisen vorzugsweise einen Naßfestmittelgehalt von ca. 0.05 - 6 Gew.-%, bevorzugt 2 - 4 Gew.-%, auf, bezogen auf die verwendete Menge an Zellulosefasermaterial.

Auf den so hergestellten, mit Naßfestmittel behandelten, porösen Trägermaterialien werden nun die Immunkomplexe durch Immunpräzipitation adsorbiert, wodurch man immunreaktive poröse Trägermaterialien erhält.

Unter immunreaktiven Trägermaterialien werden Trägermaterialien verstanden, die über die darauf präzipitierten Immunkomplexe als Immunpartner in einer immunologischen Reaktion wirken können. Dies kann über folgende Teilstrukturen der Immunkomplexe geschehen:
- über weitere immunreaktive antigene Determinanten des Antikörper oder/und Antigenanteils der Immunkomplexe,
- über weitere freie Antigenbindungsstellen eines Antikörpers der Immunkomplexe. Dabei kann dieser Antikörper im präzipitierten Immunkomplex als Antikörper- oder als Antigen-Anteil gebunden sein,
- über Gruppen, die eine spezifische, nicht immunologische Bindungswechselwirkung zu einer anderen Substanz aufweisen. Dazu gehören beispielsweise feste Enzym-Substrat-, Enzym-Coenzym-oder Enzym-Inhibitor-Wechselwirkungen.

Die Partner der Immunpräzipitation im Rahmen des erfindungsgemäßen Verfahrens sind einerseits ein Antigen, beispielsweise ein Hapten oder ein Protein, andererseits ein Antikörper. Das Antigen kann natürlich selbst ebenfalls einen Antikörper darstellen. Es kann sich hierbei sowohl um monoklonale als auch polyklonale Antikörper handeln. Unter Antikörper sind sowohl die vollständigen Antikörper als auch Fragmente hiervon, zum Beispiel Fab, Fab' bzw. (Fab')$_2$-Fragmente, zu verstehen.

Wenn ein präzipitierender Anti-Antikörper für die Fällung eingesetzt wird, muß er entsprechend ausgewählt werden. Der Anti-Antikörper selbst kann dann polyklonal oder monoklonal oder ein (Fab')$_2$-Fragment davon sein. Handelt es sich beim Anti-Antikörper um einen monoklonalen Antikörper oder ein Fragment davon, so sollten diese zweckmäßig zwei unterschiedliche Epitope am Antigen erkennen. Jedoch können auch monoklonale Antikörper bzw. deren (Fab')$_2$-Fragmente verwendet werden, die nur ein Epitop erkennen (was häufiger ist), wenn dieses Epitop auf dem Antigen mindestens zweimal vorhanden ist. Auch

4

können Mischungen von monoklonalen Antikörpern als Anti-Antikörper-Fraktion für die Immunpräzipitation eingesetzt werden.

Als zu präzipitierender immunreaktiver Stoff kann vorzugsweise auch ein Protein verwendet werden, an das ein Hapten oder Antigen gekoppelt ist. In diesem Falle wird zweckmäßig ein präzipitierender Antikörper als zweiter Partner der Immunreaktion verwendet, welcher gegen das Protein gerichtet ist. In einer weiteren Ausführungsform kann an den präzipitierenden Antikörper ein Hapten oder Antigen gekoppelt sein. Dabei kann der andere Partner der Immunreaktion unmarkiert oder mit dem gleichen oder einem anderen Hapten oder Antikörper gekoppelt sein. Alternativ ist das zu präzipitierende Protein selbst ein spezifischer Antikörper, der von einem Anti-Antikörper präzipitiert wird. An den zu präzipitierenden Antikörper kann außerdem ein Hapten oder Antigen gebunden sein. Der Präzipitierende Antikörper kann dann sowohl gegen den Antikörper, einen Teil des Antikörpers oder gegen das daran gebundene Hapten oder Antigen gerichtet sein.

Unter den Begriff Antikörper, Antigen bzw. Hapten fallen auch Substanzen, die neben der Bindungsstelle für den präzipitierenden bzw. zu präzipitierenden Partner der Immunreaktion mindestens eine weitere Bindungsstelle für einen Partner einer weiteren Immunreaktion oder einer anderen Reaktion aufweisen, bei der spezifische Wechselwirkungen, wie beispielsweise Enzym-Substrat-, Enzym-Coenzym- oder Enzym-Inhibitor-Wechselwirkungen ausgenutzt werden.

Für die Präzipitation der Immunpartner auf das Trägermaterial gibt es mehrere Möglichkeiten. Der Fachmann unterscheidet heterogene Verfahren, wie sie beispielsweise in der WO 82/02601 oder der US-A-3,888,629 beschrieben sind, und die homogenen Verfahren. Sowohl heterogene als auch homogene Verfahren zur Präzipitation sind zur Verwendung im erfindungsgemäßen Verfahren geeignet. Besonders geeignet sind das homogene 1-Schritt- und das homogene 2-Schritt-Verfahren der DE-A-34 46 636. Im ersten Verfahren wird das mit Naßfestmittel behandelte Trägermaterial mit einer Lösung, das die Immunpartner im Verhältnis des Heidelberger-Maximums und mindestens einen geeigneten Hemmstoff enthält, getränkt. Danach wird bis zu einer bestimmten Restfeuchte, beispielsweise 3 Gew %, getrocknet.

Im zweiten Verfahren wird das mit Naßfestmittel behandelte Trägermaterial zunächst mit der Lösung eines die Hemmung aufhebenden Stoffes (beispielsweise Natriumchlorid) getränkt, dann getrocknet und anschließend mit einer Lösung, das die Immunpartner im Verhältnis des Heidelberger Maximums und mindestens einen geeigneten Hemmstoff für die Präzipitation enthält, getränkt. Die Präzipitation und die Adsorption an die Oberfläche des mit Naßfestmittel behandelten Trägermaterials erfolgt während des anschließenden Trocknens.

Ein großer Vorteil des erfindungsgemäßen Verfahrens zur Herstellung von immunreaktiven porösen Trägermaterialien ist, daß Waschschritte, die aufwendig sind und außerdem Mikro- und Makroinhomogenitäten in der Belegung der Oberfläche immunreaktiver poröser Trägermaterialien durch die Immunkomplexe erzeugen, wegfallen können. Dadurch wird ein sehr homogens immunreaktives poröses Trägermaterial erzeugt.

Außerdem werden durch das erfindungsgemäße Verfahren immunreaktive poröse Trägermaterialien bereitgestellt, an denen die Immunkomplexe fest adsorbiert sind und während der Durchführung der immunologischen Tests nicht ausbluten. Die damit verbundenen Leerwerte der Messung können somit vermieden werden. Das bedeutet eine vereinfachte Testdurchführung.

Die erfindungsgemäß hergestellten immunreaktiven Trägermaterialien eignen sich jedoch auch für Tests und Trennverfahren, in denen auf einen oder mehrere Waschschritte aus verfahrensbedingten Gründen nicht verzichtet werden kann. Dadurch, daß die Immunkomplexe sehr fest an der Oberfläche des Trägermaterials haften, wird das Mitauswaschen und somit der Verlust an Immunkomplexen vor dem oder während des Tests bzw. des Trennverfahrens verringert. Das hat zur Folge, daß es möglich ist, für den Test oder das Trennverfahren bei gleichbleibender Immunkapazität des immunreaktiven Trägermaterials weniger Immunkomplexe einzusetzen.

Die Möglichkeit, erfindungsgemäß ein sehr homogenes immunreaktives poröses Trägermaterial herstellen zu können, erlaubt dessen Anwendung unabhängig von dessen Form und Größe. Die mit Naßfestmitteln behandelten Trägermaterialien können nicht nur wie beschrieben in papierähnlicher Form, sondern z. B. auch in anderen geometrischen Formen, beispielsweise in Säulen- oder Blockform ausgestaltet werden.

Auch zur Herstellung mit Naßfestmittel behandelter Trägermaterialien mit diesen anderen unterschiedlichen geometrischen Formen können die Naßfestmittel nicht nur schon dem Faserbrei zugegeben werden, sondern man kann auch zunächst dem Trägermaterial eine beliebige geometrische Form geben und es anschließend mit einer Lösung des Naßfestmittels in oben angegebener Weise tränken und trocknen.

Die Anwendung eines erfindungsgemäß hergestellten immunreaktiven porösen Trägermaterials kann insbesondere im Rahmen eines heterogenen Immunoassays zur qualitativen oder quantitativen Bestimmung eines Analyten (z. B. Hapten, Antigen, Antikörper) erfolgen. Beispielsweise wird dazu ein Hapten (Hp) oder ein Antigen (Ag) bzw. ein Antikörper (Ak), der in einer Probe wie Pufferlösung, Serum, Plasma, Urin, Kulturüberstand u. a. enthalten ist, mit einem markierten Bindungspartner (B) versetzt. Hierfür kommen u. a. Antikörper, Antikörper-Fragmente sowie Liganden in Frage, welche spezifisch mit dem Hapten oder Antigen reagieren, bzw. Hapten oder Antigen. Als Markierung kann z. B. ein Enzym, Fluoreszenz-Label oder Radioisotop verwendet werden. Die Menge des zugegebenen Bindungspartners kann je nach Testführung molmäßig sowohl im Überschuß als auch im Unterschuß zu dem in der Probe vorhandenen Hapten, Antigen oder Antikörper vorliegen.

Diese Mischung wird für eine konstante Zeit inkubiert, während der sich die Komplexe Hp-B, Ag-B bzw. Ak-B bilden. Nach Ablauf dieser Zeitspanne liegen im Reaktionsgemisch drei Spezies vor, nämlich der Komplex, bestehend aus Hapten, Antigen bzw. Antikörper und Bindungspartner (Hp-B, Ag-B, Ak-B), freies Hapten oder Antigen bzw. Antikörper (Hp, Ag, Ak) und freier Bindungspartner (B).

Die Trennung dieser Spezies erfolgt in einem zweiten Schritt mittels Immunosorption. Dazu wird das Gemisch auf das erfindungsgemäß hergestellte immunreaktive, poröse Trägermaterial aufgetragen, auf welches je nach gewählter immunologischer Meßmethode entweder das nachzuweisende Hapten bzw. Antigen oder der hiergegen gerichtete Antikörper präzipitiert ist.

Als immunologisches Meßverfahren eignet sich für die Bestimmung von Haptenen insbesondere das sogenannte IEMA-Verfahren:

- Das immunreaktive Trägermaterial mit präzipitiertem Hapten bindet den freien, nicht aber den mit Hapten abgesättigten Bindungspartner. Folglich enthält der Überstand bzw. das Eluat der Festphase den mit dem Hapten der Probe abgesättigten Bindungspartner. Die quantitative Bestimmung des Haptens erfolgt nunmehr über die Markierung des Bindungspartners.

Für die Bestimmung eines Antigens ist das sogenannte Sandwich-Verfahren bevorzugt:

- Hierzu wird ein immunreaktives Trägermaterial eingesetzt, auf das ein gegen das zu bestimmende Antigen gerichteter Antikörper präzipitiert ist. Dieser bindet den Komplex aus Antigen und Bindungspartner, nicht jedoch den freien Bindungspartner. Reste des freien Bindungspartners werden durch Waschen entfernt. Der quantitative Nachweis des Antigens erfolgt über die Markierung des Bindungspartners.

Eine andere Anwendung sind kompetitive Immuntests:

- Der Einsatz des erfindungsgemäß hergestellten immunreaktiven Trägermaterials kann so erfolgen, daß die Probe, die den Analyten (Hapten, Antigen oder Antikörper) enthält, mit einer konstanten Menge an markiertem Analyten versetzt wird. Die Markierung kann z. B. ein Enzym, ein Fluoreszenz-Label, Radioisotop und anderes sein. Diese Mischung wird auf das immunreaktive Trägermaterial gegeben. Auf dieser ist ein immunologischer Partner präzipitiert, der gegen den Analyten gerichtet ist. Die Mischung wird auf dem immunreaktiven Trägermaterial eine bestimmte Zeit inkubiert. Während dieser Zeit konkurrieren sowohl unveränderter Analyt als auch markierter Analyt um die Bindungsstellen des immunologischen Partners auf dem immunreaktiven Trägermaterial. Je mehr Analyt in der Probe vorhanden ist, desto weniger markierter Analyt wird von dem Immunreaktiven Trägermaterial gebunden und umgekehrt. Am Ende der Inkubationsphase wird die Flüssigkeit aus dem porösen immunreaktiven Trägermaterial entfernt, z. B. durch Zentrifugieren. Bestimmt wird dann die Menge des markierten Analyten entweder in der freien Phase oder die Menge des an die Festphase gebundenen markierten Analyten.

- Eine weitere Anwendung der erfindungsgemäß hergestellten immunreaktiven Trägermaterialien kann so erfolgen, daß der Analyt (Hapten, Antigen oder Antikörper) mit einer bekannten Menge an markiertem immunologischen Partner, der gegen den Analyten gerichtet ist, versetzt wird. Mögliche Arten der Markierung wurden weiter oben beschrieben. Diese Mischung wird entweder eine bestimmte Zeit inkubiert und dann auf das poröse immunreaktive Trägermaterial gegeben oder alternativ sofort nach Mischung auf das poröse immunreaktive Trägermaterial gegeben. Wenn der Analyt ein Hapten ist, enthält das immunreaktive Trägermaterial das nachzuweisende Hapten oder ein Derivat davon, ebenfalls in präzipitierter Form. Wurde die erste Mischung vor Auftragung auf das Trägermaterial inkubiert, so bindet noch freier markierter immunologischer Partner an das immunreaktive poröse Trägermaterial. Wurde die Mischung sofort auf das immunreaktive Trägermaterial gegeben, so konkurrieren der Analyt aus der Probe und der an das immunreaktive Trägermaterial fixierte Analyt um die Bindungsstellen des markierten immunologischen Partners. Am Ende der Inkubationsphase wird die Probeflüssigkeit von dem immunreaktiven porösen Trägermaterial getrennt und die Menge des markierten immunologischen Partners entweder in der flüssigen Phase oder auf dem immunreaktiven porösen Trägermaterial bestimmt.

Die nach dem erfindungsgemäßen Verfahren hergestellten immunreaktiven porösen Trägermaterialien können als Matrixvliese bei Immuntests eingesetzt werden.

Beispielhaft seien genannt:

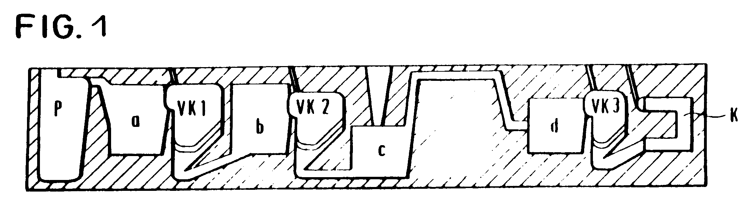

1. Die Verwendung in Einsatzelementen gemäß EP-A-0 167 171 oder EP-B-0 073 513 (beispielsweise Figur 1).

2. Die Anwendung in einem Immunteststreifen. Hier wird die Flüssigkeit, in welcher der Analyt gelöst ist, durch Kapillarkräfte von einem Vlies zum nächsten transportiert.

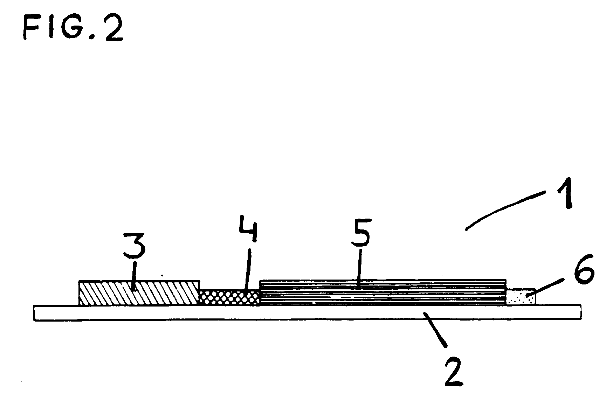

Der Aufbau eines einfachen Teststreifens ist in Fig. 2 abgebildet.

Der Teststreifen 1 besteht aus einer Trägerschicht 2 und darauf befestigten Vliesen 3, 4, 5 und 6. Die Flüssigkeit wird auf das Vlies 3 aufgegeben und löst aus Vlies 4 erforderliche Reagenzien, beispielsweise ein markiertes Antigen, Hapten oder Antikörper ab. Vlies 5 ist ein erfindungsgemäßes immunreaktives poröses Trägermaterial. Im Vlies 6 schließlich erfolgt die spektroskopische Messung der Markierung. Bei geeigneter Wahl der Markierung kann beispielsweise eine Farbänderung auch visuell beurteilt werden. Solche Immunteststreifen können auch im Rahmen von automatischen Testführungen eingesetzt werden.

Die folgenden Beispiele erläutern die Erfindung weiter:

## Beispiel 1

Herstellung von mit Naßfestmittel behandelten Trägermaterialien durch Zugabe eines Naßfestmittels in den Faserbrei

a) Als Ausgangsmaterialien dienen:

1000 l Wasser

2,4 kg Polyester (1.7 dtex, Schnittlänge 6 mm, absolut trocken; Schwarzwälder Textil Werke)

0,6 kg Sulfitzellstoff (Typ FW absolut trocken; Bayerische Zellstoff GmbH)

0,018 kg Luresin$^R$ (BASF AG, entspricht 3 Gew.-% bezogen auf Sulfitzellstoff)

Als Papiermaschine wird eine handelsübliche Schrägsiebmaschine verwendet. Die Fasermaterialien werden aufgeschlagen, gemahlen und mit Wasser und Naßfestmittel aufgeschäumt. Der entstandene Faserbrei wird auf ein Schrägsieb gepumpt. Während die Flüssigkeit abfließt, werden die Fasern auf der Sieboberfläche zurückgehalten und zum Trockner transportiert. Die Trocknung findet bei 125° C statt, bis eine Feuchtigkeit von 1.4 - 2.4 Gew. % erreicht ist. Die Absaug- und die Transportgeschwindigkeit werden so gewählt, daß ein Material einer Dichte von 0.33 g/cm$^3$ und einer Dicke von 0.6 mm entsteht.

b) Analog zu Beispiel 1 a) wird ein Trägermaterial aus folgenden Ausgangsmaterialien hergestellt:

1000 l Wasser

1 kg Sulfitzellstoff (Typ FW absolut trocken; Bayerische Zellstoff GmbH)

0,5 kg Zellwolle Viskose (Typ DAB7, absolut trocken; 1.7 dtex, Schnittlänge 6 mm; Rohtex Textil GmbH)

1 kg Polyamid (absolut trocken, 2.2 dtex, Schnittlänge 4 mm; Schwarzwälder Textil Werke)

1 kg Polyester (absolut trocken, 1.5 dtex, Schnittlänge 6 mm; Schwarzwälder Textil Werke)

1,5 kg Polyacrylnitril (absolut trocken, 3.3 dtex, Schnittlänge 4 mm; Schwarzwälder Textil Werke)

0.24 kg Etadurin$^R$ N76-Lösung (Akzo Chemie, 12.5 Gew.-% Etadurin$^R$N76, das entspricht 0.03 kg Etadurin trocken = 3 Gew.-% bezogen auf Sulfitzellstoff)

c) Analog zu Beispiel 1 a) wird ein Vlies aus folgenden Ausgangsmaterialien hergestellt:

1000 l Wasser

3 kg Polyester (1.7 dtex, Schnittlänge 6 mm, absolut trocken; Schwarzwälder Textil Werke)

2 kg Linters (absolut trocken Typ 1471/941 der Peter Tenning GmbH)

0.48 kg Etadurin$^R$ N76-Lösung (12.5 Gew.-% Etadurin$^R$N76, das entspricht 0.06 kg Etadurin trocken = 3 Gew.-% bezogen auf Linters).

## Beispiel 2

Herstellung von mit Naßfestmitteln behandelten Trägermaterialien durch Tränken des Trägermaterials mit einer Lösung eines Naßfestmittels

Als Ausgangsmaterial dient ein Trägermaterial aus 60 % Polyester und 40 % Sulfitzellstoff (Typ FW absolut trocken; Bayerische Zellstoff GmbH, Dichte 0.3 g/cm$^3$, Flüssigkeitsaufnahme 600 ml/m$^2$, Flächengewicht 200 g/m$^3$, Dicke 0.6 mm). Das Trägermaterial wird mit einer wässrigen Lösung von 4 g/l Etadurin$^R$ N76, das entspricht 3 Gew.-% bezogen auf Sulfitzellstoff) an einer Imprägniermaschine getränkt und anschließend bei 100° C auf eine Restfeuchte von 3 Gew.-% mit Umluft getrocknet.

Beispiel 3

Gewinnung weiterer Ausgangsmaterialien zur Herstellung immunreaktiver poröser Trägermaterialien und zur Testführung

A) Herstellung von Polyhapten, bestehend aus Kaninchen-IgG und daran gebundenem T3 bzw. T4

Die Herstellung von Polyhaptenen ist Stand der Technik. So kann z. B. ein Digoxin-Polyhapten über reaktive unsymmetrische Dicarbonsäureester/aktivierte Haptenester und deren Bindung an ein Trägerprotein gewonnen werden.

Die Herstellung von T3 und T4-Polyhapten erfolgt beispielsweise durch direkte Kupplung der $NH_2$-Gruppen von Hormon und Protein mittels Bis-Imidaten (EP-A 0 078 952) oder durch die Carbodiimid-Reaktion (Aherne et al., Brit. J. Clin. Pharm. 3, 56 (1976)) oder die "gemischte-Anhydrid"-Reaktion (Erlanger et al., Methods in Immunology and Immunochemistry, ed. Williams and Chase, Acad. Press (New York 1967) p. 149 ff). Alternativ kann die $NH_2$-Funktion von T3 oder T4 in einem ersten Schritt mit der Acetyl-, Trifluoracetyl-, t-Butoxicarbonyl- oder der Benzyl-oxycarbonyl-Gruppe geschützt werden. Anschließend wird die Carboxyl-Funktion in einen aktivierten Ester überführt, z. B. N-hydroxysuccinimidester, N-hydroxyphthalimidester, N-hydroxybenztriazolester. Ein Beispiel für ein so aktiviertes T4 ist beschrieben in EP-A 0 108 400, Beispiel 3. Umsetzung mit dem Trägerprotein, beispielsweise Kaninchen-IgG, ergibt das Polyhapten.

Die Auswahl der Trägerproteine unterliegt keinen Einschränkungen, sofern nur ein entsprechender "fällender" Antikörper zur Verfügung steht bzw. hergestellt werden kann.

B) Herstellung von Antikörper gerichtet gegen den Fc-Teil des Kaninchen-IgG

Kaninchen-Schlachtserum wurde einer Ammonsulfat-Fällung unterzogen. Nach Passage über DEAE-Cellulose und Papain-Spaltung nach R. R. Porter, Biochem. J. 73, 119 - 126 (1959) Gelfiltration über Sephadex G 100 und Ionenaustauschchromatographie über DEAE-Cellulose nach Literaturvorschrift (K. Malinowski und W. Manski in "Methods in Enzymology"; J.J. Langone und H. van Vunakis eds., Academic Press, Vol. 73 (1981) pp. 418 - 459) erhält man die Fc-Fragmente des Kaninchen-IgG's als Immunogene. Mit diesem Immunogen wurden Schafe immunisiert und das entsprechende Antiserum gewonnen.

Antiserum, gerichtet gegen den Fc-Teil des Kaninchen-IgG, wurde über Ammoniumsulfat-Fällung und Passage über DEAE-Cellulose zur IgG-Fraktion aufgereinigt.

Für die anschließende immunsorptive Aufreinigung von Antikörper wurde als Träger das "Affinitätsadsorbers, Glutardialdehyd aktiviert" von Boehringer Mannheim (Best.-Nr. 665525) verwendet. Die Durchführung der Immunosorption erfolgte wie in der Arbeitsanleitung zum Affinitätsadsorbens beschrieben.

C) Gewinnung von Konjugat aus Antikörper gegen T4 und β-D-Galaktosidase

Antikörper gegen T4 wurden an β-D-Galactosidase gekoppelt nach der Vorschrift von T. Kitiwaga in Enzyme Immunoassay (Eds. Ishikawa, Kawai, Migai; Igaku Shoin Tokyo/New York (1981) pp. 81 - 89)

Beispiel 4

Herstellung von immunreaktivem porösem Trägermaterial (homogenes 1-Schritt-Verfahren)

200 mg Polyhapten, bestehend aus Kaninchen IgG und daran gebundenem T3, und 700 mg Antikörper, gerichtet gegen den Fc-Teil des Kaninchen IgG's (Heidelberger Maximum) werden getrennt in je 1 Liter Lösung aus 0.05 M Essigsäure und 0.5 % Natriumchlorid aufgenommen. Nach 30 Minuten werden die Lösungen vereinigt. Das gemäß Beispiel 1 a) mit Naßfestmittel behandelte Trägermaterial wird mit dieser Lösung getränkt und bei 70 ° C auf eine Restfeuchte von 3 Gew.-% getrocknet.

Beispiel 5

Herstellung von immunreaktivem porösen Trägermaterial (homogenes 2-Schritt-Verfahren

a) Trägermaterial, das nach der Vorschrift des Beispiels 1 a) mit Naßfestmittel behandelt wurde, wird mit einer 0.5 Gew.-%igen Natriumchloridlösung getränkt und bei 70 ° C auf eine Restfeuchte von 3 % getrocknet.

b) 200 mg Polyhapten, bestehend aus Kaninchen IgG und daran gebundenem T4, und 700 mg Antikörper, gerichtet gegen den Fc-Teil des Kaninchen-IgG's (Heidelberger Maximum) werden in je 1 Liter 0.05 M Essigsäurelösung gelöst. Nach 30 Minuten werden die Lösungen vereinigt. Das mit Naßfestmittel behandelte Trägermaterial wird mit dieser Lösung getränkt und bei 70 °C auf eine Restfeuchte von 3 Gew.-% getrocknet.

Beispiel 6

Herstellung von immunreaktivem porösem Trägermaterial (mit Albumin)

100 mg Albumin und 350 mg polyklonaler Antikörper, gerichtet gegen Albumin, werden getrennt in je 1 l Lösung aus 0.05 M Essigsäure und 0.5 % Natriumchlorid aufgenommen. Nach 30 Minuten werden die Lösungen vereinigt. Das mit Naßfestmittel behandelte Trägermaterial wird mit dieser Lösung getränkt und bei 70 °C auf eine Restfeuchte von 3 Gew.-% getrocknet.

Beispiel 7

Bestimmung der Matrixbindung M (Bindungsfestigkeit)

Nach Beispiel 1 a) werden drei Sorten von Trägermaterialien hergestellt. Dabei werden als Naßfestmittel Etadurin[R] N76 (Akzo Chemie) (Vlies 1), Madurit[R] (Typ MW 150, Hoechst AG) (Vlies 2) und Tylose[R] (Typ CBR 200, Hoechst AG) (Vlies 3) in einer Menge von 3 Gew.-%, bezogen auf Sulfitzellstoff, verwendet. Als Vergleich dient ein Trägermaterial gemäß Beispiel 1 a), bei dessen Herstellung aber auf die Verwendung von Naßfestmitteln verzichtet worden war (Vlies 4).

Aus jedem dieser Trägermaterialien wird nach Beispiel 4 durch Präzipitation und Trocknung ein immunreaktives poröses Trägermaterial hergestellt.

Die Bindungsfestigkeit der Immunkomplexe auf jedem der immunreaktiven Träger wird in einem Einsatzelement gemäß Fig. 1 unter Verwendung eines Zentrifugalanalysators gemäß EP-A-132510 bestimmt. Ein Einsatzelement ähnlichen Aufbaus ist beschrieben in EP-B-0073513.

Dazu wird aus den vier verschiedenen immunreaktiven porösen Trägermaterialien je ein Matrixvlies (Vliese 1 - 4) der gleichen, den Anforderungen des Einsatzelementes entsprechenden Größe geschnitten.

Bestückung der Einsatzelemente nach Fig. 1

Kammern a, b, P, K, VK1, VK2, VK3:  leer
Kammer c:  1 Matrixvlies aus immunreaktivem, porösem Trägermaterial
Kammer d:  1 Substratvlies
(Vlies getränkt mit Chlorphenolrot-$\beta$-D-galaktosid (CPR-G), 20 mmol/l, HEPES 50 mmol/l, pH 7.5)

Testführung:

1. Es werden 50 µl einer Lösung von 200 U/l Konjugat aus $\beta$-D-Galaktosidase als Markierung und Antikörper gegen T4 in Phosphatpuffer 50 mmol/l, pH 7.5 (mit Crotein C[1] 0,5 %, Tween 20[2] 0,3 %) in Kammer c pipettiert.
2. Man inkubiert 5 Minuten lang. Dabei bildet sich ein Immunkomplex Y aus dem als Antigen wirkenden Immunkomplex X von Polyhapten, bestehend aus Kaninchen IgG und daran gebundenem T4, und Antikörper, gerichtet gegen den Fc-Teil des Kaninchen IgG, mit dem als Antikörper wirkenden Konjugat aus Antikörper gegen T4 und $\beta$-D-Galaktosidase als Markierung.
3. Es wird in einem Zentrifugalanalysator gemäß EP-A-0 132 510 20 Sekunden lang zentrifugiert, wobei Immunkomplexe Y, die nicht fest an den Träger gebunden sind, zusammen mit der Lösung aus Kammer c in Kammer d transportiert werden. Alle Komplexe Y, die Kammer c wegen schlechter Bindung an die Matrix verlassen, weisen wegen der Inkubation in Schritt 2) $\beta$-Galaktosidase-Aktivität auf. Die Flüssigkeit löst dabei die in Kammer d befindlichen Reagenzien und nimmt sie in Kammer VK3 mit.
4. Sobald die Zentrifugation beendet ist, verläßt die Lösung Kammer VK3.

5. Während der folgenden erneuten Zentrifugation fließt die Lösung in die Küvette K. Dort wird die $\beta$-Galaktosidase-Aktivität A (mE/min) in der Lösung durch absorptionspektroskopische Messung der Änderung der Konzentration des aus dem Substrat CPR-G gebildeten Produkts bei 576 nm ermittelt. Die gemessene Enzymaktivität ist ein Maß für die Menge an Immunkomplex Y, die sich wegen mangelhafter Bindungsfestigkeit von dem immunreaktiven porösen Träger gelöst hat. Findet sich keine Enzymaktivität (A = 0 mE/min), so ist die Bindung des Immunkomplexes Y an die Matrix vollständig (Leerwert L = 0 %)

Die Enzymaktivität wird mit dieser Testführung für vier Einsatzelemente bestimmt, deren Kammern c jeweils mit einem der Matrixvliese 1, 2, 3 oder 4 bestückt wurden.

Nullwert

Als Nullwert $E_o$ dient das Ergebnis der Messung der Enzymaktivität A, wenn bei sonst gleicher Testführung in Kammer c kein immunreaktives poröses Trägermaterial eingelegt wird. Da sich deshalb kein Immunkomplex Y bilden kann und so keine Bindung an eine Matrix stattfindet, entspricht der Nullwert einem Leerwert L von 100 %.

Der Nullwert bei einem Leerwert von 100 % kann genauso bestimmt werden, indem in Kammer c in mit Naßfestmitteln behandeltes Trägermaterial eingelegt wird, welches das Konjugat nicht oder nur in sehr geringem Ausmaß adsorbiert.

Den Leerwert L (%) der Vliese errechnet man mit folgender Gleichung:

$$L = \frac{E}{E_O} \cdot 100$$

Die für die Matrixvliese 1 - 4 erhaltenen Werte sind in Tabelle 1 zusammengestellt.

Tabelle 1

| Matrixvlies | Naßfestmittel | Enzymaktivität A(mE/min) | Leerwert L (%) |
|---|---|---|---|
| - | - | 2000 | 100 |
| 1 | Etadurin | 104 | 5.2 |
| 2 | Madurit | 36 | 1.8 |
| 3 | Tylose | 14 | 0.7 |
| 4 | - | 249 | 12.4 |

Der Leerwert läßt sich also durch Verwendung von erfindungsgemäßem immunreaktivem porösen Trägermaterial deutlich senken.

**Patentansprüche**

1. Immunreaktives poröses Trägermaterial, bestehend aus einem porösen Trägermaterial und darauf präzipierten Immunkomplexen, dadurch gekennzeichnet, daß das poröse Trägermaterial aus Fasern besteht und mit einem oder mehreren Naßfestmitteln behandelt ist.

2. Trägermaterial gemäß Anspruch 1, dadurch gekennzeichnet, daß das poröse Trägermaterial aus einem Gemisch von Zellulose- und Kunststoffasern besteht.

3. Trägermaterial gemäß Anspruch 2, dadurch gekennzeichnet, daß der Kunststoffaseranteil bis zu 95 Gew.-% beträgt.

4. Trägermaterial gemäß einem der Ansprüche 1 bis 3, dadurch gekennzeichnet, daß das oder die Naßfestmittel ausgewählt werden aus der Gruppe der Epichlorhydrinharze, Melaminformaldehydharze, Harnstofformaldehydharze, Polyethylenimine und Carboxymethylcellulosen.

EP 0 312 907 B1

5. Verfahren zur Herstellung eines immunreaktiven porösen Trägermaterials gemäß Anspruch 1 durch Präzipitieren eines Immunkomplexes auf ein poröses Trägermaterial , das aus Fasern besteht, dadurch gekennzeichnet, daß das poröse Trägermaterial mit einem oder mehreren Naßfestmitteln behandelt und darauf anschließend die Immunkomplexe präzipitiert werden.

6. Verfahren zur Herstellung eines immunreaktiven porösen faserförmigen Trägermaterials gemäß Anspruch 1, dadurch gekennzeichnet, daß das oder die Naßfestmittel einem Faserbrei zugesetzt werden, dieser anschließend getrocknet und hierauf die Immunkomplexe präzipitiert werden.

7. Verfahren gemäß Anspruch 5 oder 6, dadurch gekennzeichnet, daß das poröse Trägermaterial aus einem Gemisch von Zellulose- und Kunststoffasern besteht.

8. Verfahren gemäß einem der Ansprüche 5 bis 7, dadurch gekennzeichnet, daß das oder die Naßfestmittel ausgewählt werden aus der Gruppe der Epichlorhydrinharze, Melaminformaldehydharze, Harnstofformaldehydharze, Polyethylenimine und Carboxymethylcellulosen.

9. Verwendung eines immunreaktiven porösen Trägermaterials gemäß einem der Ansprüche 1 bis 4 bei heterogenen Immunoassays.

**Claims**

1. Immune reactive porous carrier material consisting of a porous carrier material and immune complexes precipitated thereon, characterised in that the porous carrier material consists of fibres and is treated with one or more wet-strength agents.

2. Carrier material according to claim 1, characterised in that the porous carrier material consists of a mixture of cellulose and synthetic resin fibres.

3. Carrier material according to claim 2, characterised in that the proportion of synthetic fibre material amounts to up to 95 wt.%.

4. Carrier material according to one of claims 1 to 3, characterised in that the wet-strength agent or agents are selected from the group of epichlorohydrin resins, melamine-formaldehyde resins, urea-formaldehyde resins, polyethyleneimines and carboxymethylcelluloses.

5. Process for the production of an immune reactive porous carrier material according to claim 1 by precipitation of an immune complex on a porous carrier material, which consists of fibres, characterised in that the porous carrier material is treated with one or more wet-strength agents and the immune complexes are subsequently precipitated thereon.

6. Process for the production of immune reactive porous fibrous carrier material according to claim 1, characterised in that the wet-strength agent or agents are added to a fibre pulp, this is subsequently dried and the immune complexes are precipitated thereon.

7. Process according to claim 5 or 6, characterised in that the porous carrier material consists of a mixture of cellulose and synthetic resin fibres.

8. Process according to one of claims 5 to 7, characterised in that the wet-strength agent or agents are selected from the group of epichlorohydrin resins, melamine-formaldehyde resins, urea-formaldehyde resins, polyethyleneimines and carboxymethylcelluloses.

9. Use of an immune reactive porous carrier material according to one of claims 1 to 4 in heterogenous immunoassays.

**Revendications**

1. Matériau de support poreux immunoréactif, constitué d'un matériau de support poreux et d'un immunocomplexe précipité sur celui-ci, caractérisé en ce que le matériau de support poreux est

11

constitué de fibres et qu'il est traité avec un ou plusieurs agents qui augmentent la résistance à l'état mouillé.

2. Matériau de support selon la revendication 1, caractérisé en ce que le matériau de support poreux est constitué d'un mélange de fibres cellulosiques et synthétiques.

3. Matériau de support selon la revendication 2, caractérisé en ce que la proportion de fibres synthétiques peut aller jusqu'à 95% en poids.

4. Matériau de support selon l'une quelconque des revendications 1 à 3, caractérisé en ce que le ou les agents qui augmentent la résistance à l'état mouillé sont choisis dans le groupe des résines d'épichlorhydrine, des résines mélamine-formaldéhyde, des résines urée-formaldéhyde, des polyéthylèneimines et de la carboxyméthylcellulose.

5. Procédé de préparation d'un matériau de support poreux immunoréactif selon la revendication 1, par précipitation d'un immunocomplexe sur un matériau de support poreux, constitué de fibres, caractérisé en ce que le matériau de support poreux est traité avec un ou plusieurs agents qui augmentent la résistance à l'état mouillé et qu'ensuite, l'immunocomplexe est précipité sur ce support.

6. Procédé de préparation d'un matériau de support poreux fibreux immunoréactif selon la revendication 1, caractérisé en ce que le ou les agents qui augmentent la résistance à l'état mouillé sont introduits dans une bouillie de fibres, que est ensuite séchée, et l'immunocomplexe est précipité dessus.

7. Procédé selon la revendication 5 ou 6, caractérisé en ce que le matériau de support poreux est constitué d'un mélange de fibres cellulosiques et synthétiques.

8. Procédé selon l'une quelconque des revendications 5 à 7, caractérisé en ce que le ou les agents qui augmentent la résistance à l'état mouillé sont choisis dans le groupe des résines d'épichlorhydrine, des résines mélamineformaldéhyde, des résines urée-formaldéhyde, des polyéthylèneimines et de la carboxyméthylcellulose.

9. Utilisation d'un matériau de support poreux immunoréactif, selon l'une quelconque des revendications 1 à 4, pour des dosages immunologiques hétérogènes.

FIG. 1

FIG.2